# FASCICULE DE BREVET EUROPEEN

(11) **EP 0 758 384 B1**
(45) Date de publication et mention de la délivrance du brevet: **09.04.2003**
(21) Numéro de dépôt: 95918044.9
(22) Date de dépôt: 21.04.1995
(51) Int. Cl.: C12N 15/30, C12N 15/86, C07K 14/44, C07K 16/20, C12N 5/10, C12Q 1/68, C12N 7/01, A61K 39/008, A61K 39/395, A61K 35/70

(54) **PROTEINE MAJEURE DE LEISHMANIES, ACIDE NUCLEIQUE CODANT POUR CETTE PROTEINE ET LEURS APPLICATIONS**
LEISHMANIA HAUPTPROTEIN, DAFÜR KODIERENDE NUKLEINSAÜRE UND VERWENDUNGEN DAVON
LEISHMANIA MAJOR PROTEIN, NUCLEIC ACID CODING THEREFOR, AND USES THEREOF

(30) Priorité: 02.05.1994 FR 9405313
(43) Date de publication de la demande: 19.02.1997
(73) Titulaire: CENTRE NATIONAL DE LA RECHERCHE SCIENTIFIQUE, 75016 Paris Cédex (FR)
(72) Inventeur: GLAICHENHAUS, Nicolas, F-06100 Nice (FR); MOUGNEAU, Evelyne, F-06100 Nice (FR); ALTARE, Frédéric, F-98000 Monaco (FR); CALBO, Sébastien, F-34970 Maurin (FR); SOLDERA, Sylvelie, F-06560 Valbonne (FR)
(74) Mandataire: Pernez, Helga
(86) Numéro de dépôt international: FR9500529
(87) Numéro de publication internationale: WO95030006

(56) Documents cités:
- FR-A- 2 615 103
- JOURNAL OF IMMUNOLOGY., vol. 144, no. 3, 1 Février 1990 BALTIMORE US, pages 1075-1079, PHILLIP SCOTT ET AL. 'Protection against Leishamnia major in BALB/c mice by adoptive transfer of a T cell clone recognizing a low molecular weight antigen released by promastigotes' cité dans la demande
- INFECTION AND IMMUNITY, vol. 60, no. 4, Avril 1992 WASHINGTON US, pages 1368-1374, ARVE OSLAND ET AL. 'Isolation and characterization of recombinant antigens from Leishmania aethopica that react with human antibodies'

## Description

La présente invention concerne une nouvelle protéine majeure de leishmanies utile pour la préparation d'un vaccin contre les leishmanioses chez les mammifères et plus particulièrement chez l'homme et le chien.

Les leishmanioses sont des maladies endémiques d'origine parasitaire, dont les agents causales, les leishmanies, sont des protozoaires flagellés appartenant à l'ordre des kinetoplastidés et à la famille des trypanosomatidés. Un grand nombre de types de leishmanies sont susceptibles d'infecter l'homme; selon le type considéré et les facteurs génétiques et immunologiques de l'hôte, elles sont à l'origine de manifestations cliniques très différentes. On distingue ainsi les leishmanioses cutanées, muco-cutanées et viscérales. Certaines de ces maladies, telles que les leishmanioses viscérales, sont fatales en l'absence de traitement, d'autres, comme les formes muco-cutanées, sont très défigurantes.

Bien qu'en pleine expansion chez l'homme dans les régions tropicales, les leishmanioses sont essentiellement des maladies du chien, lequel est le principal réservoir du parasite. Dans les régions développées, la progression de la leishmaniose canine est liée incontestablement à l'accroissement du nombre de chiens domestiques. Il n'est pas pratiqué aujourd'hui d'abattage systématique des chiens malades ou asymptomatiques et le traitement utilisé à base de dérivés antimoniés s'avère d'autant plus décevant qu'une première cure entraîne dans certains cas une guérison apparente de l'animal qui rechute souvent quelques mois plus tard.

On sait par ailleurs que l'immunodépression liée aux virus de l'immunodéficience humaine favorise la survenue d'infections qualifiées d'opportunistes. Le parasite opportuniste vit en équilibre avec l'hôte sain et n'entraîne aucune manifestation pathologique. Il a pour caractéristique de devenir pathogène en cas d'altération des défenses immunitaires. L'incidence élevée de la leishmaniose viscérale chez les patients infectés par le VIH amène à considérer qu'il s'agit bien là d'une infection opportuniste. L'étude poussée de la leishmaniose constitue donc aujourd'hui un enjeu majeur en terme de santé publique.

Le cycle de ces parasites est relativement simple et l'on distingue deux formes, dénommées promastigote et amastigote, qui infectent des hôtes spécifiques et qui diffèrent tant par la morphologie que par le métabolisme :
- La forme promastigote, munie d'un long flagelle, se développe dans le tractus digestif de diptères hématophages vecteurs de la maladie, appelés communément phlébotomes et appartenant aux genres *Phlebotomus, Lutzmya* et *Psycholopygus*.
- La forme amastigote, pourvue d'un très court flagelle, se multiplie de manière quasi exclusive dans les macrophages de certains mammifères.

Ainsi, lorsqu'un phlébotome infecté pique un mammifère pour se nourrir, il lui injecte des promastigotes; ceux-ci sont rapidement phagocytés par les macrophages et se transforment en amastigotes. Après plusieurs cycles de multiplication, chaque macrophage peut contenir des dizaines de parasites. On suppose qu'à ce stade, les macrophages éclatent et libèrent les amastigotes qui peuvent propager l'infection en colonisant des macrophages sains. Si le repas sanguin d'un phlébotome est effectué sur un mammifère infecté, il peut être contaminé par des macrophages parasités; les amastigotes ainsi captés vont alors se transformer en promastigotes dans la lumière du tube digestif des insectes, assurant la perpétuation du cycle.

Le phlébotome transmet ainsi la maladie du chien au chien et du chien à l'homme. Ce qui explique que maladies humaines et animales sont étroitement liées.

En outre, la plupart des leishmanies pathogènes pour l'homme peuvent également infester les rongeurs couramment utilisés au laboratoire, lesquels développent des pathologies souvent très proches de celles rencontrées chez l'homme et constituent donc des modèles expérimentaux adaptés pour tester le pouvoir vaccinant d'antigènes. La sensibilité aux leishmanies des différentes lignées de souris syngéniques est sous contrôle génétique et dépend essentiellement des immunités innées et acquises; ainsi les souris C57BI/6 et C3H sont résistantes à l'infection par diverses espèces de leishmanies et d'autres, telles BALB/c sont particulièrement sensibles.

Les premiers essais de vaccination chez l'homme contre la leishmaniose viscérale ou cutanée ont utilisé des souches parasites tuées par chauffage ou par irradiation; plusieurs essais cliniques ont été effectués avec différentes souches comme *Leishmania major, Leishmania mexicana* ou *Leishmania brasiliensis.* Les résultats des essais réalisés avec *L. Major,* en présence de BCG comme adjuvant, montrent que des quantités très importantes de parasites doivent être injectées pour obtenir des niveaux de protection suffisants (WHO Special Programme for Research and Training in Tropical Diseases Progress Report 1991-1992).

Il a aussi été proposé d'utiliser des fractions polypeptidiques de leishmania présentant des propriétés immunogènes, par exemple dans les Demandes de Brevet Français publiées sous les numéros 2 615 103, 2 612 779 et 2 577 140.

Les inconvénients majeurs limitant l'utilisation de ces antigènes protecteurs pour la préparation de vaccins résident d'une part, dans les difficultés de production de ces fractions protéiques par extraction de tissus, et d'autre part, dans le risque potentiel, bien que celui-ci soit toujours minime, d'effets indésirables liés à la mise en oeuvre de produits directement issus des parasites.

Les développements de la biologie moléculaire permettent aujourd'hui de nouvelles approches de recherche et de production de protéines de leishmanies.

Ainsi, plusieurs protéines ont été récemment identifiées et testées à titre de vaccins sur des modèles de souris :
- La protéine la plus étudiée est la glycoprotéine gp63 décrite par Etges R., Bouvier J. et Bordier C. (*J. Biol. Chem*. 1986; 261:9098-9101). Le pouvoir vaccinant de cette protéine a été testé sans succès chez la souris BALB/c (Russel D.G., Alexander J.; *J. Immunol* 1988; 140:1274-1279). Le clonage de la gp63 (Button L.L., McMaster W.R., *J. Exp. Med.* 1988; 167:724-729) a permis de préparer un peptide synthétique, lequel, associé à un adjuvant approprié, a permis d'obtenir une protection significative (Jardim A., Alexander J.; *J. Exp. Med.* 1990; 172:645-648 . Russo DM., Turco S.J., Burns J.M. Jr., Reed SG.; *J. Immunol.* 1992; 148:202-207 . Russo DM., Burns JM. Jr., Carvalho EM.; *J. Immunol*. 1991; 147:3575-3580).
- Une autre glycoprotéine de surface, d'un poids moléculaire de 46 kD, a également été clonée (Lohman KL., Langer PJ., McMahon-Pratt D.; *Proc. Natl. Acad. Sci. USA* 1990; 87:8393-8397) et testée. Cette protéine est capable de conférer chez la souris BALB/c une protection contre l'infection par le parasite (Champsi J., McMahon-Pratt D.; *Infect. Immun*. 1988; 52:3272-3279).
- Un antigène de surface original a également été décrit comme susceptible de conférer une protection; il s'agit du lipophosphoglicanne aussi dénommé "LPG", un glycoconjugé exprimé en abondance à la surface des promastigotes et qui joue un rôle de récepteur lors de l'invasion des macrophages (Handman E., Goding JW.; *EMBO J.* 1985; 4:329-336). L'immunisation de souris BALB/c avec l'antigène LPG dans de l'adjuvant de Freund induit une excellente protection contre l'infection par *L. Major,* apparemment selon un mécanisme "cellule T dépendant" (Mitchell GF., Handman E.; *Parasitol Today* 1985; 1:61-63 . Jardim A., Tolson DL., Turco SJ., Pearson TW., Olafson RW.; *J. Immunol*. 1991; 147:3538-3544).
- Un antigène soluble de leishmania dénommé "SLA" issu de promastigotes et injecté intrapéritonéalement avec l'adjuvant *C. parvum*, semble également protéger les souris BALB/c contre *L. Major* (Scott P., Pearce E., Natovitz P., Sher A.; *J. Immunol.* 1987; 139:221-227). La séparation de ce produit brut par HPLC a révélé une sous-fraction unique responsable de l'immunisation (Scott P., Pearce E., Natovitz P., Sher A.; *J. Immunol.* 1987; 139:3118-3125). Une protéine d'environ 10 kD a été identifiée à partir d'un clone de cellules T protecteur issu d'une souris immunisée avec cette sous-fraction (Scott P., Caspar P., Sher A.; *J. Immunol*. 1990; 144:1075-1079).

Une autre approche pour la mise au point d'un vaccin contre la leishmaniose a consisté à isoler des lignées ou des clones de lymphocytes T protecteurs à partir de souris ayant résisté à l'infection par le parasite; il a été observé que l'injection de ces clones à des souris susceptibles est capable d'induire une protection contre l'infection (Scott, P., Natowitz, P., Coffman, R., Pearce, E. & Sher, A.; *J. Exp. Med.* 1988; 168:1675-1684). L'article de Scott P. et al. cité précédemment (Scott P., Caspar P., Sher A.; *J. Immunol.* 1990; 144:1075-1079), décrit l'obtention et la caractérisation d'un tel clone, dénommé "Th9.1-2".

Il a par ailleurs été observé par Locksley R. et al. (Reiner, S., Wang, Z.-E., Hatam, F., Scott, P., Locksley, R.; *Science* 1993; 259:1457-1460) que l'infection de souris par le parasite *L. Major* provoque la prolifération d'une sous-population de lymphocytes CD4+ porteurs d'un récepteur particulier qui utilise les régions Vβ4 et Vα8; ces auteurs ont montré que c'est cette même sous-population de lymphocytes qui prolifère chez les souris susceptibles et chez les souris résistantes suggérant ainsi que le même antigène parasitaire constitue la cible principale des lymphocytes T chez les deux types de souris; ils ont par ailleurs montré que le clone de lymphocyte T Th9.1-2 utilise également les régions Vβ4 et Vα8.

D'un point de vue purement académique, ce sont les réponses immunitaires se développant aux cours d'infections murines dues à *L. Major* qui ont été le plus étudiées. Ces travaux montrent que les lymphocytes T CD4+ jouent un rôle fondamental dans la résolution mais aussi dans l'aggravation des lésions (Titus, R., Ceredig, R., Cerottini, J.-C., Louis, J.; *J. Immunol.* 1985, 135:2108-2114). Tant chez les souris résistantes que susceptibles, on note respectivement une expression préférentielle de lymphocytes T CD4+ TH1 et TH2. Les expériences réalisées sur la souris BALB/c, très sensible à *L. major,* ont permis de confirmer les corrélations entre :
- le phénotype résistant et la réponse lymphocytaire de type TH1, et,
- le phénotype susceptible et la réponse lymphocytaire de type TH2.

Les lymphocytes T CD4+ TH1 et TH2 sont caractérisés par le panel de lymphokines qu'ils sécrètent, et c'est précisément par le biais de ces lymphokines que les différents types de lymphocytes T CD4+ limitent ou favorisent l'infection par le parasite.

Les lymphocytes TH1 produisent de l'interleukine-2 et de l'interféron γ, lequel active les macrophages et les rend leishmanicides (Locksley, R. M. and P. Scott.; *Immunology Today*, 1991; 58-61).

Les lymphocytes TH2 produisent de l'interleukine-4 ou de l'interleukine-10, qui peuvent d'une part, bloquer l'activation des macrophages par l'interféron γ, et d'autre part, inhibent la production des lymphokines synthétisées par les lymphocytes TH1 (Locksley, R. M. and P. Scott.; *Immunology Today,* 1991; 58-61).

Si la présence de ces lymphokines semble une condition nécessaire au développement d'un phénotype donné, elle n'est pas suffisante puisque le traitement de souris génétiquement résistantes ou sensibles avec respectivement de l'interleukine-4 ou de l'interféron γ n'a pas d'effet à long terme sur des lésions. Cependant, l'injection d'interleukine-4 à des souris résistantes infectées induit une réponse transitoire de type TH2 et l'injection d'interféron γ à des souris sensibles infectées provoque la synthèse d'interféron γ endogène et diminue la production d'interleukines 4 et 5 (Locksley, R. M. and P. Scott.; *Immunology Today,* 1991; 58-61).

Par ailleurs, des études récentes ont montré que l'interleukine 12 (IL-12) jouait un rôle déterminant dans le développement des lymphocytes T de type Th1 (Hsieh, C.-S., Heimberger, A.B., Gold, J.S., O'Garra, A. & Murphy, K.M.; *Proc. Natl. Acad. Sci. U.S.A*., 1993; 89: 6065). En fait, l'administration d'IL-12 recombinante à des souris susceptibles de la souche BALB/c provoque la guérison de ces animaux lorsqu'ils sont infectés par le parasite *Leishmania major* (Heinzel, F.P., Schoenhaut, D.S., Rerko, R.M., Rosser, L.E., Gately, M.K.; *J. Exp. Med*., 1993; 177:1505). De plus, la neutralisation de l'IL-12 conduit à un développement de la maladie chez des souris génétiquement résistantes à l'infection par ce parasite (Sypek, J.P., Chung, C.L., Mayor, S.E.H., Subramanayam, J.M., Goldman, S.J., Sieburth, D.S., Wolf, S.F., Schaub, R.G; *J. Exp. Med.,* 1993; 177:1797). Cet effet peut être la conséquence indirecte de l'induction d'interféron γ et peut être régulé négativement par IL-10 (Heinzel, F.P., Schoenhaut, D.S., Rerko, R.M., Rosser, L.E., Gately, M.K.; *J. Exp. Med*., 1993; 177:1505).

On sait en outre que l'activation des lymphocytes T CD4+ nécessite l'intervention de cellules accessoires capables de présenter des antigènes parasitaires dans le contexte de molécule de classe II spécifiées par le complexe majeur d'histocompatibilité. En effet, les antigènes protéiques ne sont pas reconnus sous leur forme native par les lymphocytes CD4+, mais requièrent un traitement préalable consistant le plus souvent en une protéolyse ménagée génératrice de peptides immunogènes capables de se lier aux molécules de classe II (*Fundamental Immunology,* third edition, Raven Press, 1993).

Les cellules présentatrices d'antigènes qui sont impliquées dans le développement des lymphocytes T CD4+ spécifiques de leishmanies n'ont pas encore été caractérisées, mais plusieurs candidats sont envisagés, tels que les lymphocytes B, les macrophages infectés ou non infectés (Prina, E., C. Jouanne, S. D. S. Lao, A. Szabo, J. G. Guillet and J. C. Antoine.; *J. Immunol*., 1993; 151:2050-2061). Les macrophages jouent en effet un rôle ambivalent dans les processus liés à l'infection par les leishmanies; ils représentent à la fois les cellules hôtes indispensables à la croissance des parasites et les cellules qui, une fois activées, vont développer des mécanismes leishmanicides (Locksley, R. M. and P. Scott.; *Immunology Today,* 1991; 58-61)

Les Inventeurs ont mis à profit l'ensemble des connaissances académiques précédentes pour entreprendre une stratégie de recherche efficace permettant d'identifier de nouvelles protéines du parasite *L. Major,* utiles pour la préparation d'un vaccin contre différentes souches du parasite *Leishmania* chez l'homme et l'animal.

La stratégie suivante a été mis en oeuvre :
- Il a tout d'abord s'agit d'identifier un antigène de *L. Major* reconnu par le clone Th9.1-2.; pour ce faire une technique de clonage par expression a été utilisée. Cette technique a consisté à construire une banque d'ADN complémentaire du parasite *L. Major* dans un vecteur procaryote, à exprimer les protéines recombinantes dans la bactérie et à purifier lesdites protéines sur des colonnes d'affinité; puis, les protéines recombinantes ainsi obtenues, ont été testées pour leur capacité à induire la sécrétion d'interleukine-2 par les cellules d'un hybridome, dénommé ci-après LMR16.2, préparé à partir du clone T protecteur TH9.1-2. Parmi les 50 lots de 5000 clones qui ont ainsi été fabriqués et testés, les Inventeurs ont identifié un clone exprimant une protéine recombinante de 24 Kd qui sera dénommée dans ce qui suit "protéine p24".
- Dans un second temps, le plasmide dont est porteur le clone correspondant à la protéine p24 a été étudié, et un insert de 582 paires de bases a été identifié. Les Inventeurs ont observé que cet insert s'hybride dans des conditions de forte stringence avec deux espèces d'ARN messagers de la forme promastigote de *L. Major* de 1500 et 1800 nucléotides. La taille de cet insert étant inférieure à celles des ARNm correspondant, les Inventeurs ont cloné l'ADN complémentaire complet correspondant à la protéine p24. Il a été ainsi caractérisé un acide nucléique de 1070 paires de bases comprenant une séquence de 936 nucléotides codant pour une protéine de 312 acides aminés et d'un poids moléculaire de 35 Kd qui sera dénommée dans ce qui suit "protéine p35".

Des expériences d'hybridation moléculaires en conditions stringentes ont également montré que des ARN messagers homologues étaient présents chez d'autres souches du parasite *Leishmania (L. donovani, L. amazonensis, L. chagasi, L. braziliensis, L. guyanensis, L. panamensis, L. tropica, L. mexicana*). On connaît en effet un grand nombre de types de leishmanies, mais il a été montré, par exemple dans la Demande de Brevet Européen publiée sous le numéro 197 062, que les différentes espèces de leishmanies possédaient des déterminants antigéniques communs induisant la protection d'anticorps neutralisants et donc susceptibles d'être utilisés dans la vaccination contre les différentes espèces de parasites.

La présente invention a donc pour support l'identification d'une nouvelle protéine majeure de leishmanies utile principalement pour la préparation d'un vaccin contre la leishmaniose chez les mammifères, et plus particulièrement chez l'homme et le chien.

C'est pourquoi, l'invention a tout d'abord pour objet, une protéine comprenant la séquence de 312 acides aminés représentée dans la SEQ ID NO:1 de la liste de séquences en annexe, ou une forme modifiée ou une partie de celle-ci dès lors qu'elle est reconnue par des anticorps anti-p35. De tels anticorps sont présents chez les animaux infectés par le parasite *L. Major,* mais peuvent aussi être préparés au laboratoire.

Les techniques permettant la préparation d'anticorps monoclonaux ou polycolonaux dirigés contre la protéine p35 ou une forme modifiée ou une partie de celle-ci sont bien connues de l'homme du métier.

Les anticorps polyclonaux sont préparés par immunisation en injectant à un animal, par exemple un lapin ou une souris, une quantité appropriée de la protéine p35 préparée soit par extraction et purification de matériel biologique de *L. Major,* soit par synthèse chimique de la séquence de 312 acides aminés représentée dans la SEQ ID NO:1 de la liste de séquences en annexe, soit encore par expression de l'ADN codant pour la protéine p35 dans un hôte.

Les anticorps monoclonaux sont produits par tout hybridome préparé selon les méthodes de fusion cellulaire entre des cellules spléniques, activées *in vitro* par la protéine p35 ou provenant d'un animal immunisé contre ladite protéine, et des cellules d'une lignée de cellules myélomateuses.

Ces anticorps, ainsi que ceux préparés à partir d'un fragment de la protéine p35 seule ou associée à une molécule porteuse sont utiles pour le dépistage immunologique *in vitro* d'une leishmaniose, et font donc également partie de l'invention.

La protéine de l'invention peut être aussi un fragment polypeptidique ou peptidique de la séquence en acides aminés représentée dans la SEQ ID NO:1 de la liste de séquences en annexe. Il s'agit alors de fragments représentant plus de 10 acides aminés consécutifs de ladite séquence. L'invention englobe, également les protéines, polypeptides et peptides modifiés ne se distinguant des précédents que par substitutions et/ou addition et/ou suppression de un ou plusieurs acides aminés, dès lors qu'ils conservent au moins 40 % d'homologie avec la séquence dont ils dérivent.

Une protéine particulière selon l'invention comprend la partie N terminale de la séquence de la protéine p35 représentée dans la SEQ ID NO:1 de la liste de séquences en annexe. A titre spécifique, on peut citer la protéine p24 dont la séquence est délimitée par les acides aminés situés aux positions 143 et 312 dans la SEQ ID NO:1. Comme indiqué précédemment, l'invention concerne aussi les peptides représentant plus de 10 acides aminés consécutifs de ladite séquence et notamment ceux recouvrant la séquence de la protéine p24. Les Inventeurs ont identifié parmi ceux-ci, un peptide de 18 acides aminés délimités par les acides aminés situés aux positions 156 et 173 dans la SEQ ID NO:1 et portant l'épitope reconnu par l'hybridome LMR16.2 et par le clone Th9.1-2; ce peptide répond à la formule suivante :
Ile Cys Phe Ser Pro Ser Leu Glu His Pro Ile Val Val Ser Gly Ser Trp Asp.

L'invention concerne aussi un conjugué présentant des propriétés antigéniques, constitué par une protéine conforme à l'invention couplée, par exemple par covalence, à une molécule porteuse telle que la sérum albumine.

Une protéine conforme à l'invention peut être préparée par synthèse chimique ou peut être produite par génie génétique dans différentes cellules hôtes selon la technique des ADN recombinants.

La synthèse chimique consiste par exemple à condenser plusieurs fragments préalablement formés par couplages successifs des différents acides aminés, en prenant soin de protéger toutes les fonctions réactives portées par les acides aminés exceptées les fonctions amine et carboxylique engagées dans la liaison peptidique formée lors de la condensation.

La présente invention s'intéresse plus particulièrement à la synthèse dirigée par l'acide nucléique codant pour la protéine p35 ou une forme modifiée ou une partie de celle-ci.

C'est pourquoi, l'invention a également pour objet un acide nucléique codant pour la protéine p35 ou une forme modifiée ou une partie de celle-ci.

Un acide nucléique selon l'invention comprend la séquence nucléotidique représentée dans la SEQ ID NO:1 de la liste de séquences en annexe, ou une forme modifiée ou une partie de celles-ci dès lors qu'elle est capable de s'hybrider avec ladite séquence ou avec son complémentaire.

L'invention concerne en effet tout fragment de l'acide nucléique représentée dans la SEQ ID NO:1 de la liste de séquences en annexe, ou tout acide nucléique ne se distinguant des précédents que par des substitutions et/ou addition et/ou suppression de un ou plusieurs nucléotides, du fait notamment de la dégénérescence du code génétique, dès lors que ces acides nucléiques peuvent s'hybrider avec la séquence nucléotidique représentée dans la SEQ ID NO:1 de la liste de séquences en annexe, dans les conditions de stringence définies ci-après :
- traitement de pré-hybridation du support sur lequel on fixe l'acide nucléique représenté dans la SEQ ID NO:1 de la liste de séquences en annexe, à 42°C, pendant 120 minutes avec une solution de pré-hybridation constituée de : formamide 50%, NaCl 900 mM, Na Citrate 90 mM, SDS 1%, ADN de thymus de veau 100 µg/ml, solution de Denhart 5X;
- hybridation avec l'acide nucléique complémentaire pendant 18 heures à 42°C dans une solution constituée de : formamide 50 %, NaCl 900 mM, Na Citrate 90 mM, SDS 1 %, ADN de thymus de veau 100 µg/ml, solution de Denhart 5X, sulfate de dextran 40%;
- 3 lavages de 30 minutes à 42°C avec une solution constituée de : NaCl 300 mM, NaCitrate 30 mM, SDS 0,1%.

Parmi les fragments d'acide nucléiques définies ci-dessus, l'invention concerne tout particulièrement ceux constituant des sondes moléculaires ou ceux comprenant environ 10 paires de base et constituant des amorces oligonucléotidiques utiles à une réaction de polymérisation en chaîne. Ces sondes et ces amorces constituent des outils avec lesquels l'homme du métier est à même de cloner des séquences nucléotidiques homologues chez différentes souches du parasite *Leishmania* qui entre dans le cadre de la présente invention.

La séquence nucléotidique représentée dans la SEQ ID NO:1 de la liste de séquences en annexe comporte une région 5' non codante de 71 nucléotides. Le codon ATG situé aux positions 72, 73, 74 ouvre un cadre de lecture de 936 nucléotides. Le codon stop TAA situés aux positions 1008, 1009, 1010 est suivi d'une région 3' non codante de 63 nucléotides.

La séquence d'un acide nucléique conforme à l'invention comprend la séquence d'ADN délimitée par les nucléotides situés aux positions 72 et 1010 de la séquence nucléotidique représentée dans la SEQ ID NO:1 de la liste de séquences en annexe, codant pour la protéine p35 de 312 acides aminés. La séquence d'ADN délimitée par les nucléotides situés aux positions 498 et 1010, code pour la protéine p24 correspondant à la partie N terminale de la protéine p35.

L'invention a aussi pour objet tout acide nucléique recombinant qui comprend au moins un acide nucléique codant pour tout ou partie de la protéine p35, associé avec un promoteur et/ou un terminateur de transcription reconnu par les enzymes de la cellule hôte dans laquelle ledit acide nucléique recombinant est introduit. Avantageusement l'acide nucléique recombinant selon l'invention est introduit dans une cellule hôte au moyen de vecteurs; le vecteur ainsi que les signaux de contrôle de l'expression de l'acide nucléique recombinant sont choisis en fonction de la cellule hôte, eucaryote ou procaryote, dans lequel il est placé. Le vecteur peut être un plasmide mais aussi un vecteur viral capable d'infecter la cellule hôte. Les techniques permettant le clonage et l'expression d'un acide nucléique recombinant dans différentes cellules hôtes sont connues de l'homme du métier; elles seront illustrées dans la description des exemples qui suit, étant entendu que d'autres vecteurs et cellules hôtes peuvent être utilisés.

L'invention concerne donc également les cellules eucaryotes ou procaryotes transformées contenant au moins un acide nucléique recombinant décrit ci-dessus. Ces cellules transformées sont utiles pour la production d'une protéine selon l'invention. Ces techniques de recombinaison et de transformation permettent la production desdites protéines incomplètes ou fusionnées à d'autres protéines afin de favoriser une meilleure expression ou une excrétion desdites protéines hors de la cellule hôte.

L'invention concerne plus particulièrement les virus recombinants vivants, avantageusement inactivés, tels que les virus de la vaccine, les baculovirus, utiles pour la préparation de compositions vaccinantes.

L'invention concerne aussi une méthode de dépistage *in vitro* d'une infection par un parasite responsable de la leishmaniose dans un prélevement biologique. Une telle méthode peut être réalisée grâce à la protéine de l'invention, ou un anticorps dirigé contre cette protéine, ou encore à l'aide des sondes décrites précédemment.

La méthode de dépistage *in vitro* mettant en oeuvre des anticorps, préférentiellement monoclonaux, comprend les étapes suivantes :
- la mise en contact d'un anticorps reconnaissant spécifiquement la protéine p35 ou un fragment de cette protéine conforme à l'invention, avec un prélèvement biologique d'un sujet susceptible d'être infecté par un parasite responsable de leishmaniose, dans des conditions permettant la formation d'un complexe immunologique entre ledit anticorps et les protéines antigèniques contenus dans le prélèvement;
- la détection dudit complexe par tout moyen approprié connu de l'homme du métier.

Avantageusement les anticorps mis en oeuvre dans cette méthode sont marqués, par exemple de manière radioactive ou au moyen de réactif enzymatique.

Des variantes ou perfectionnements de la méthode précédente sont bien connus de l'homme du métier; il s'agit par exemple de la méthode ELISA, de la détection des complexes immunologiques à l'aide d'immunoglobulines marquées, de techniques immunoenzymatiques par compétition, ou encore de l'utilisation de plusieurs anticorps dont un au moins est spécifique de tout ou partie de la protéine p35.

Le prélèvement biologique sur lequel est réalisé le dépistage selon l'invention est préférentiellement le sang.

L'invention a en conséquence pour objet les trousses de diagnostic pour la mise en oeuvre du procédé précédent. A titre d'exemple, une telle trousse comprend notamment :
- une quantité déterminée d'un anticorps conforme à l'invention,
- un milieu approprié à la formation de complexes immunologiques entre les protéines antigéniques contenues dans le prélèvement analysé et ledit anticorps,
- des réactifs permettant la détection des complexes éventuellement formés,
- éventuellement des échantillons témoins.

Le dépistage *in vitro* d'une infection par un parasite responsable de la leishmaniose, selon une technique immunologique, peut aussi être réalisée à l'aide d'un agent de diagnostic constitué d'une ou plusieurs protéines de l'invention, d'un conjugué ou d'une composition antigénique conformes à l'invention.

Selon ce mode de réalisation, on met en contact l'agent de diagnostic défini ci-dessus, avec un prélèvement biologique, dans des conditions permettant la formation d'un complexe immunologique entre ledit agent de diagnostic et les anticorps anti-p35 éventuellement présents dans le prélèvement analysé, puis l'on révèle l'éventuel complexe par tout moyen approprié connu de l'homme du métier.

L'invention concerne donc aussi les composition antigéniques précédentes ainsi que les trousses pour le dépistage d'une infection par le parasite responsable de la leishmaniose, mettant en oeuvre ladite composition ou une protéine ou conjugué de l'invention, selon le procédé décrit ci-dessus. A titre d'exemple, une telle trousse comprend notamment :
- une quantité déterminée d'au moins une protéine selon l'invention ou d'un conjugué ou d'une composition antigénique conforme à l'invention,
- un milieu approprié à la formation de complexes immunologiques entre les anticorps contenus dans le prélèvement analysé et ladite composition antigénique,
- des réactifs permettant la détection des complexes éventuellement formés,
- éventuellement des échantillons témoins.

Outre le dépistage d'une infection par un parasite responsable de la leishmaniose, le but essentiel de l'invention est de permettre la conception et la fabrication de compositions vaccinantes efficaces tant chez l'homme que l'animal contre les différentes espèces de leishmanies.

Ce but est atteint grâce à des compositions pharmaceutiques utiles comme vaccins chez l'homme ou l'animal contre la leishmaniose, caractérisées en ce qu'elles comportent à titre d'agent actif au moins une protéine selon l'invention éventuellement dans un véhicule pharmaceutiquement acceptable.

L'invention concerne plus particulièrement des vaccins vivants, caractérisés en ce qu'ils contiennent des micro-organismes recombinants vivants, tels que des virus et des bactéries, qui expriment une protéine de l'invention, éventuellement présentés dans un véhicule pharmaceutiquement acceptable.

Parmi, lesdits micro-organismes recombinants vivants, l'invention envisage à titre spécifique un virus de la vaccinne recombinant vivant et le Bacille de Calmette Guérin (BCG).

Les compositions vaccinantes selon l'invention pourront comprendre outre l'agent actif conforme à l'invention un autre constituant actif capable de renforcer la protection induite par ledit agent actif de l'invention. On peut citer comme exemples de tels constiuants actifs accessoire l'Adjuvant de Freund Complet, l'Adjuvant de Freund Incomplet, le RIBI, l'interleukine 12 recombinante, des bactéries de la souche *Corynebacterium parvum.*

Ces vaccins sont sous forme administrable par exemple par injection et dans ce cas le véhicule pharmaceutiquement acceptable est aqueux.

Le processus de vaccination ainsi que les doses d'agent actif devront être adaptés au type de vaccin utilisé ainsi qu'au mammifère auquel il est administré.

D'autres avantages et caractéristique de l'invention apparaîtront au cours de la description qui suit et qui se réfère à des exemples notamment de clonage de l'acide nucléique codant pour la protéine p35, étant entendu que ces exemples ne sauraient constituer une limitation quelconque de l'objet de l'invention.

### I - Principe de la stratégie utilisée.

L'identification d'un antigène majeur du parasite *L. Major,* a été réalisée selon une méthode de clonage par expression utilisant la construction dans un vecteur procaryote d'une banque d'ADN complémentaire de parasite, l'expression des protéines recombinantes dans la bactérie et leur purification sur des colonnes d'affinité.

Ces protéines recombinantes ont ensuite été testées pour leur capacité à induire la secrétion d'interleukine-2 par les cellules d'un hybridome LMR16.2 obtenu à partir du clone T protecteur Th9.1-2.

### 1) Description du clone T protecteur Th9.1-2

Le clone Th9.1-2 a été isolé à partir d'une lignée cellulaire qui a été elle-même préparée à partir de souris de la souche BALB/c immunisées avec des parasites inactivés. Ce clone réagit avec une fraction protéique préparée à partir de promastigotes du parasite *Leishmania major* et avec des extraits solubles des parasites *Leishmania donovani, Leishmania braziliensis* et *Leishmania amazonensis*. En outre, l'injection des cellules de ce clone à des souris de la souche BALB/c protège ces souris de l'infection par le parasite *Leishmania major* (Scott, P., Caspar, P., Sher, A., *J. Immunol*. 1990, vol. **44,** pages 1075-1079).

### 2) Description de l'hybridome LMR16.2.

Cet hybridome a été obtenu en fusionnant des cellules du clone Th9.1-2 avec des cellules de lymphome mutantes, cellules BW5147 α-β- (Coligan, J.E., Kruisbeek, A., Margulies, D.H., Shevach, E.M., Strober, W., *Currents Protocols in Immunology* 1993, Greene, Editions Wiley - Interscience), déficientes pour l'expression du récepteur T.

Comme les cellules du clone Th9.1-2, les cellules de l'hybridome LMR16.2 utilisent les régions Vβ4 et Vα8 du récepteur T. En outre, ces cellules LMR16.2 secrètent de l'interleukine-2 en présence d'extraits bruts de parasite *Leishmania major* et de cellules présentatrices (cellules de rate) préparées à partir de souris BALB/c.

### II - production des protéines recombinantes.

Une banque d'ADN complémentaire a été construite en utilisant comme source d'ARN des promastigotes du parasite *Leishmania major.*

De l'ARN total a été préparé à partir de promastigotes métacycliques et utilisé pour la préparation d'ARN polyA⁺. Le vecteur utilisé pour la construction de la banque est un dérivé du vecteur pET3a (commercialisé par la société NOVAGEN) qui a été modifié de manière à introduire en aval du codon d'initiation de la traduction une séquence nucléotidique codant pour 6 résidus histidine consécutifs.

Une banque d'environ 10⁶ clones a été obtenue et ces clones ont été regroupés en lots de 5000.

Les bactéries correspondantes ont été congelées et des fractions aliquotes utilisées pour la production de protéines recombinantes.

Ces protéines ont été produites après infection des bactéries par le bactériophage CE6 (commercialisé par la société NOVAGEN) selon un protocole recommandé par le constructeur.

Les protéines recombinantes ont été purifiées sur des colonnes d'affinité (résine Ni-NTA commercialisée par la société QUIAGEN) selon un protocole recommandé par le constructeur, puis dialysées extensivement contre du milieu de culture (DMEM) selon un protocole classiquement utilisé (Ausubel, F.M., Brent, R., Kingston, R.E., Moore, D.D., Seideman, J.G., Smith, J.A., Struhl, K., *Currents Protocols in Molecular Biology* 1993, Greene, Editions Wiley - Interscience).

### III - Identification des clones positifs.

Des cellules de l'hybridome LMR16.2 ont été utilisées comme sondes pour le criblage proprement dit de la banque,

Le criblage a été effectué en incubant les échantillons contenant les protéines recombinantes (50 µl) pendant 24 heures dans les puits d'une plaque de microtitration de 96 puits (commercialisé par la Société COSTAR) avec des cellules de rate préparées à partir souris BALB/c (10⁶ cellules par puit) et les cellules de l'hybridome LMR16.2 (10⁵ cellules par puit) dans un volume total de 200 µl.

Les lots de clones positifs ont été identifiés en mesurant la présence d'interleukine-2 dans les surnageants de culture en utilisant des cellules indicatrices CTLL-2 (Coligan, J.E., Kruisbeek, A., Margulies, D.H., Shevach, E.M., Srober, W., *Currents Protocols in Immunology* 1993, Greene, Editions Wiley - Interscience) dépendantes de l'interleukine-2 pour leur croissance.

Les préparations de suspensions cellulaires, la mise en culture des cellules, la méthode de dosage de l'interleukine-2 sont des techniques classiquement utilisées (Coligan, J.E., Kruisbeek, A., Margulies, D.H., Shevach, E.M., Srober, W., *Currents Protocols in Immunology* 1993, Greene, Editions Wiley - Interscience).

### IV - Caractérisation d'un clone positif.

Le criblage de 50 lots de 5000 clones a permis d'identifier un clone, dénommé 23.12.10.33, exprimant une protéine recombinante de 24 kilodaltons (protéine p24).

Cette protéine recombinante produite après induction du clone 23.12.10.33 par infection avec le bactériophage CE6, et purifiée sur des colonnes d'affinité en billes de Nickel-agarose, induit la secrétion d'interleukine-2 par les cellules de l'hybridome LMR16.2.

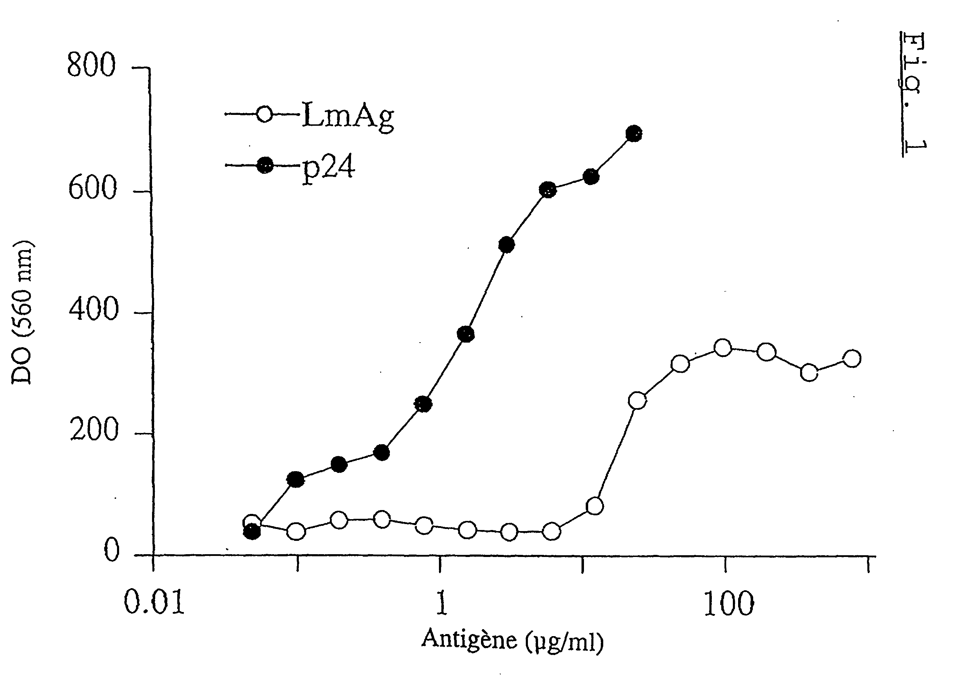

La figure 1 en annexe représente la production d'interleukine-2, exprimée en unité arbitraires OD (560 nm), par les cellules de l'hybridome LMR16.2 après incubation pendant 24 heures avec des cellules de rate de la souris BALB/c et des quantités variables de protéine p24 (p24) ou d'extraits bruts (LmAg) préparés à partir de promastigotes du parasite *Leishmania major.* La préparation des surnageants cellulaires et la méthode de dosage de l'interleukine-2 sont celles classiquement utilisées (Coligan, J.E., Kruisbeek, A., Margulies, D.H., Shevach, E.M., Srober, W., *Currents Protocols in Immunology* 1993, Greene, Editions Wiley - Interscience).

En outre, la protéine p24 induit la production d'interféron γ par les cellules du clone Th9.1-2.

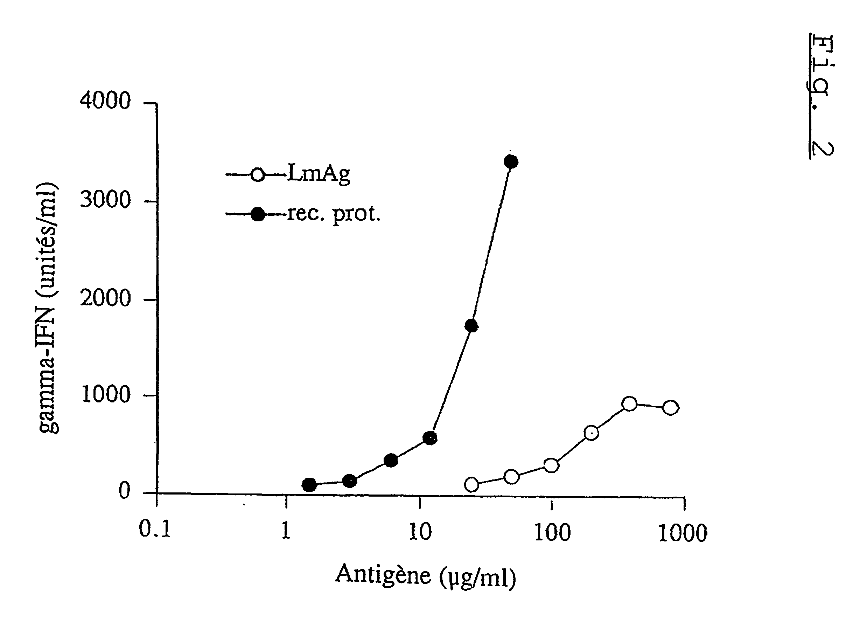

La figure 2 en annexe représente la production d'interféron γ, exprimée en unités par ml, par les cellules du clone Th9.1-2 après incubation pendant 24 heures avec des cellules de rate de souris de la souche BALB/c et des quantités variables de protéine p24 (rec.prot.) ou d'extraits bruts (LmAg) préparés à partir de promastigotes du parasite *Leishmania major.* La préparation des surnageants cellulaires et la méthode de dosage de l'interféron γ sont celles classiquement utilisées (Coligan, J.E., Kruisbeek, A., Margulies, D.H., Shevach, E.M., Srober, W., *Currents Protocols in Immunology* 1993, Greene, Editions Wiley - Interscience).

### V - Identification de l'épitope reconnu par l'hybridome LMR16.2 et par le clone TH9.1-2.

Afin d'identifier le peptide antigénique avec lequel réagit l'hybridome LMR16.2, des peptides recouvrants de 18 acide aminés ont été synthétisés à partir de la séquence de la protéine p24.

Ces peptides ont ensuite été incubés avec les cellules de l'hybridome LMR16.2 et des cellules de rate préparées à partir de souris BALB/c. Après 24 heures d'incubation, les surnageants de culture ont été récoltés et testés pour la présence d'interleukine-2.

Ces expériences ont permis d'identifier un peptide de 18 acides aminés qui stimule la sécrétion d'interleukine-2 et qui est également capable de stimuler les cellules du clone Th9.1-2 pour la sécrétion d'interféron γ.

Ce peptide de 18 acides aminés est délimité par les acides aminés situés aux positions 156 et 173 dans la SEQ ID NO:1 et répond à la formule suivante :

Ile Cys Phe Ser Pro Ser Leu Glu His Pro Ile Val Val Ser
Gly Ser Trp Asp.

### VI - L'ARN messager correspondant à la protéine recombinante p24 est fortement conservé entre différentes souches de parasite Leishmania major.

Le plasmide dont est porteur le clone 23.12.10.33 contient un insert de 582 paires de bases cloné entre les sites EcoR1 et HindIII du vecteur pET3a-d9 et dont la séquence a été déterminée.

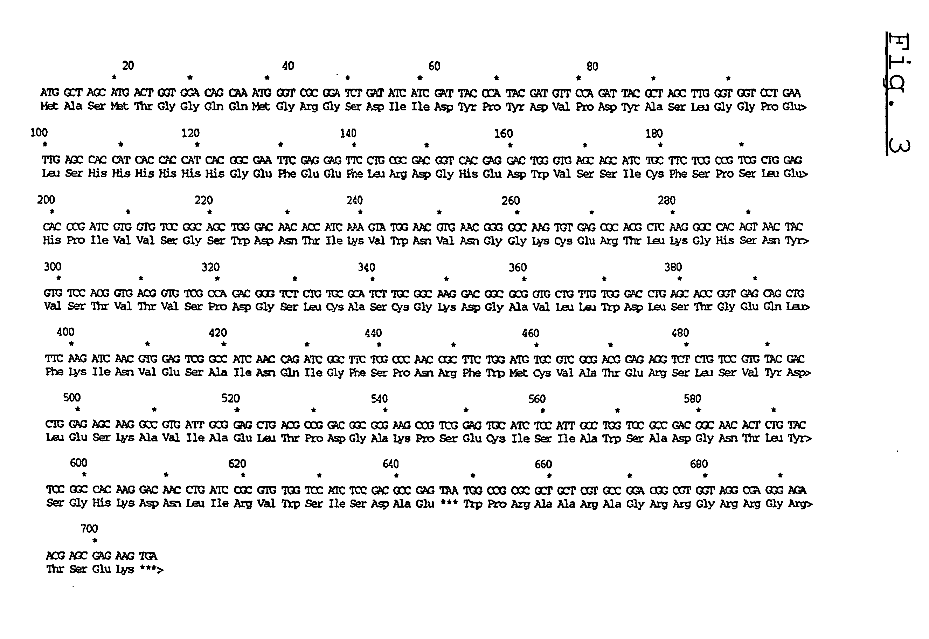

La séquence nucléotidique de cet insert est représentée à la figure 3. Cette séquence contient une phase de lecture correspondant à une protéine d'un poids moléculaire de 24 kilodaltons (216 acides aminés) se décomposant en une séquence leader de 5 kilodaltons (45 acides aminés) codée par le vecteur (séquence soulignée dans la figure 3) et une séquence de 19 kilodaltons (171 acides aminés) correspondant à la phase ouverte de l'ADN complémentaire.

L'insert du clone 23.12.10.33 hybride avec deux espèces d'ARN messagers de la forme promastigotes du parasite *Leishmania major* de 1300 et 1800 nucléotides dans des conditions de fortes stringences.

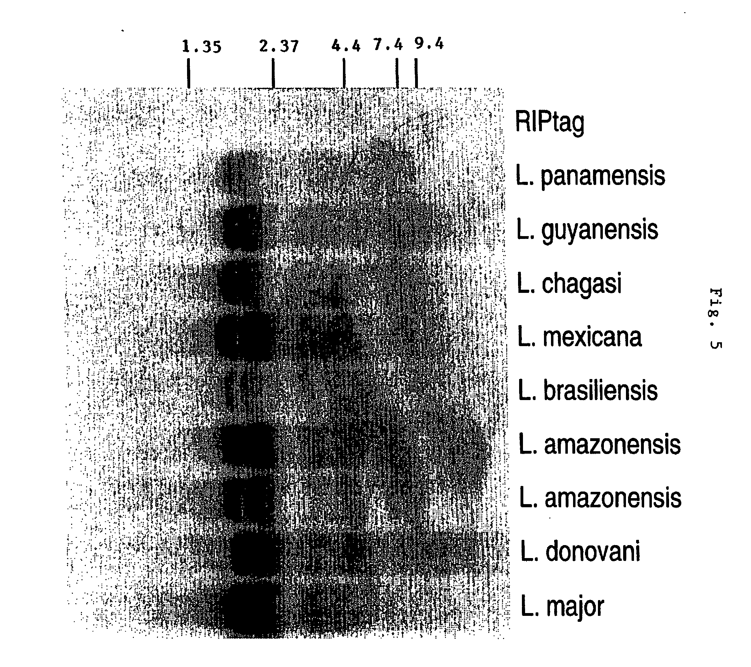

Des expériences d'hybridation moléculaire en conditions stringentes ont également montré que des ARN messagers homologues étaient présents chez d'autres souches du parasite *Leishmania* (*L. donovani, L. amazonensis, L. chagasi, L. braziliensis, L. guyanensis, L. panamensis, L. tropica, L. mexicana*). La figure 5 représente une autoradiographie obtenue après hybridation d'une sonde moléculaire radioactivement marquée préparée à partir de l'acide nucléique correspondant à la SEQ ID NO:1 avec des ARN totaux préparés à partir de promastigotes de différentes souches de Leishmania (10 µg par piste), dans les conditions de stringences suivantes : 6 X SSC, 50% formamide, 42°C. Les techniques utilisées pour la préparation des ARN, la séparation de ces ARN par électrophorèse en gel d'agarose et l'hybridation avec une sonde moléculaire sont celles classiquement utilisées (Ausubel, F.M., Brent, R., Kingston, R.E., Moore, D.D, Seideman, J.G., Smith, J.A., Struhl, K., *Currents Protocols in Molecular Biology* 1993, Greene, Editions Wiley - Interscience).

### VII - Obtention d'un ADN complémentaire complet correspondent à la protéine p24.

La taille de l'insert du clone 23.12.10.33 étant inférieure à celles des ARN messagers correspondants, un ADN complémentaire complet correspondant à cette protéine a été cloné.

Pour celà, il a été exploité une particularité des ARN messagers du parasite *Leishmania* qui est de posséder à leur extrémité 5' une séquence leader de quelques dizaines de nucléotides.

De l'ARN polyA⁺ de promastigotes du parasite *Leishmania major* a été préparé et utilisé comme matrice pour synthétiser de l'ADN complémentaire en présence de l'enzyme transcriptase réverse commercialisé par la Société BRL et d'une amorce oligonucléotidique oligo-dT 12-18 commercialisé par la Société PHARMACIA. Le protocole de synthèse de cet ADN complémentaire est celui classiquement utilisé (Ausubel, F.M., Brent, R., Kingston, R.E., Moore, D.D, Seideman, J.G., Smith, J.A., Struhl, K., *Currents Protocols in Molecular Biology* 1993, Greene, Editions Wiley - Interscience).

L'ADN complémentaire obtenu a ensuite été amplifié en utilisant la technique de la réaction de polymérisation en chaîne en présence de deux amorces nucléotidiques :
- LM8 (5'ATTACTCGGCGTCGGAGAT3')
- LM9 (5'AACTAACGCTATATAAGTATCAG3')
correspondant respectivement à une séquence localisée dans la région 3' de l'insert du clone 23.12.10.33 (LM8) et à la séquence leader des ARN messagers du parasite *Leishmania major* (LM9). Les conditions de la réaction sont celles préconisées par le fournisseurs de l'enzyme TAQ polymérase. Trente cycles ont été effectués (1 minute à 94°C, 2 minutes à 55°C et 3 minutes à 72°C).

Un fragment d'ADN de 1070 paires de bases a ainsi été obtenu et sous cloné entre les sites EcoR1 et Sma1 du vecteur Bluescript commercialisé par la Société STRATAGENE.

La séquence nucléotidique de cet insert a été déterminée et la séquence polypeptidique correspondante a été déduite de la séquence nucléotidique.

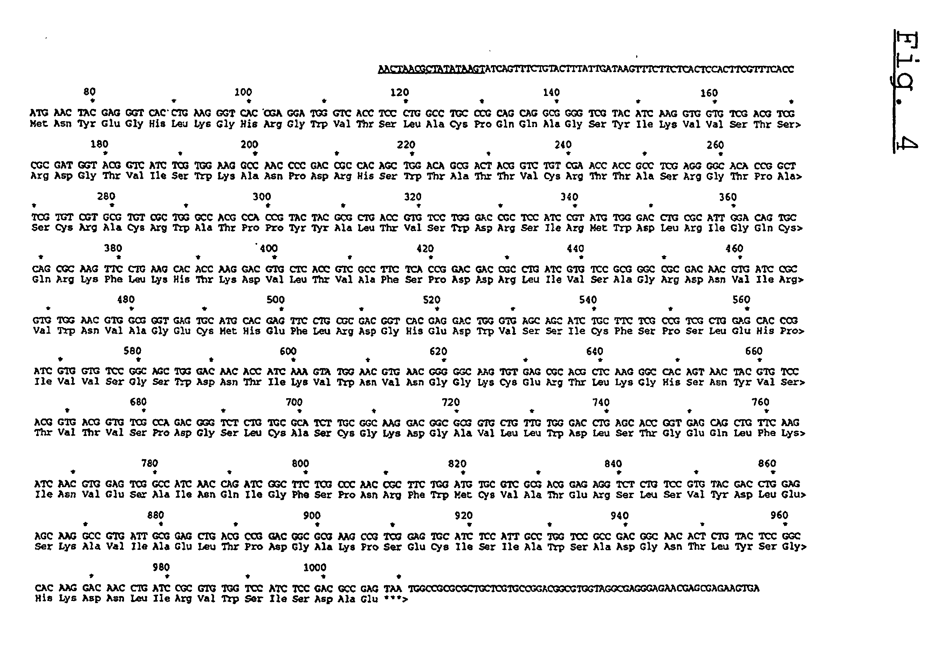

Ces séquences sont représentées à la figure 4 ainsi que dans la SEQ ID NO:1 de la liste de séquences en annexe.

Cette séquence nucléotidique comporte une région 5' non traduite de 71 nucléotides suivie d'une séquence codante de 936 nucléotides et d'une séquence 3' non codante de 63 nucléotides. La séquence codante correspond à une protéine de 312 acides aminés d'un poids moléculaire attendu de 35 kilodaltons (protéine p35).

### VIII - La protéine p35 est largement conservée entre différentes espèces du parasite Leishmania.

La protéine p35 est largement conservée entre différentes espèces du parasite *Leishmania* comme l'atteste les résultats de l'expérience d'hybridation moléculaire représentée à la figure 5.

En conséquence, la séquence nucléotidique codant pour la protéine p35 permet de définir, par hybridation dans des conditions de faible stringence, les acides nucléiques codant pour des protéines, plypeptides et peptides présentant des homologies avec la protéine p35.

Les conditions d'hybridation de faible stringence indiquées ci-dessus sont par exemple les suivantes :
- traitement de pré-hybridation du support sur lequel on fixe l'acide nucléique représenté dans la SEQ ID NO:1 de la liste de séquences en annexe, à 42 °C, pendant 120 minutes avec une solution de pré-hybridation constituée de : formamide 50%, NaCl 900 mM, Na Citrate 90 mM, SDS 1%, ADN de thymus de veau 100 µg/ml, solution de Denhart 5X;
- hybridation avec l'acide nucléique complémentaire pendant 18 heures à 42 °C dans une solution constituée de : formamide 30%, NaCl 900 mM, Na Citrate 90 mM, SDS 1%, ADN de thymus de veau 100 µg/ml, solution de Denhart 5X, sulfate de dextran 40%;
- 3 lavages de 30 minutes à 37 °C avec une solution constituée de NaCl 300 mM, Na Citrate 30 mM, SDS 0,1 %.

La séquence nucléotidique codant pour la protéine p35, ou une forme modifiée ou une partie de celle-ci, peut ainsi être utilisée comme sonde moléculaire pour le clonage de séquence homologue chez d'autres souches du parasite *Leishmania*. Par exemple, une sonde moléculaire préparée à partir de la séquence nucléique définie par la SEQ ID NO:1 peut être utilisée pour cribler une banque d'ADN complémentaire préparée à partir d'ARN de promastigotes de différentes souches de leishmania en utilisant des techniques bien connues de l'homme du métier (Ausubel, F.M., Brent, R., Kingston, R.E., Moore, D.D, Seideman, J.G., Smith, J.A., Struhl, *K., Currents Protocols in Molecular Biology* 1993, Greene, Editions Wiley - Interscience).

De même, des oligonucléotides appropriés s'hybridant avec la séquence nucléique codant pour la protéine p35, peuvent être utilisées dans des processus d'amplification par PCR pour le clonage de séquences nucliques homologues d'autres souches du parasite Leishmania selon les techniques classiquements utilisées (Ausubel, F.M., Brent, R., Kingston, R.E., Moore, D.D, Seideman, J.G., Smith, J.A., Struhl, K., *Currents Protocols in Molecular Biology* 1993, Greene, Editions Wiley - Interscience).

### IX - Production de la protéine p35 ou d'une partie de celle-ci.

La synthétise de la protéine p35 ou de formes tronquées de celle-ci peut être réalisée selon différentes techniques classiquement utilisées en microbiologie et en biologie moléculaire à partir de tout ou partie de la séquence nucléotidique codant pour la dite protéine.
1) La production d'un acide nucléique peut être réalisée par exemple par l'une des méthodes suivantes :
   - Technique de PCR pour amplifier à partir d'ADN complémentaire de promastigotes du parasite Leishmania major un fragment d'ADN correspondant à tout ou partie de la séquence de la la SEQ ID NO:1 en annexe.
   - Synthèse d'un oligonucléotide hybridant avec la séquence de la la SEQ ID NO:1 en annexe et utilisation pour le criblage d'une banque d'ADN complémentaire ou d'une banque génomique préparée à partir de la forme promastigote du parasite *Leishmania major.*
2) La synthèse de la protéine peut être réalisée par exemple par l'une des méthodes suivantes :
   - Clonage de la séquence nucléotidique dans un vecteur d'expression procaryote (pET, pGST...), production de la protéine recombinante et purification sur des colonnes d'affinité (Hochuli, E., 1989, vol. 12, pages 87-89, In "Genetic Engineering, Principle and Methods, J. K. Setlow Ed, Plenum Press, New York). Des techniques similaires ont été utilisées pour produire et purifier différentes protéines recombinantes, par exemple l'aldolase du parasite *Plasmodium Falciparum* (Dôbeli, H., Trecziak, A., Gillesen, D., Matile, H., Srivastava, I.K., Perrin, L.H., Jakob, P.E. & Certa, U., 1990 *Molecular and Biochemical Parasitology,* vol. 41, pages 259-268) ou la transcriptase reverse du virus HIV (Le Grice, S.F.J. & Gruenionger,-Leitch, F., 1990 *Eur. J. Biochem*., vol. 187, pages 307-314).
   - Clonage de la séquence nucléotidique dans un vecteur d'expression eucaryote (vecteur vaccine, bacculovirus, levure) et purification de la protéine correspondante à partir du surnageant cellulaire et/ou d'extraits protéiques préparés après lyse des cellules. Une approche similaire a été utilisée dans de très nombreux cas, en particulier pour le clonage de gènes dans des vecteurs vaccines recombinants (Janknecht, R. & de Martynoff, G., Lou, J., Hipskind, R.A., Nordheim, A. & Stunnenberg, H.G., *1991 PNAS,* vol. 88, pages 8972-8976).
   - Préparation de peptides et polypeptides synthétiques (Coligan, J.E., Kruisbeek, A., Margulies, D.H., Shevach, E.M., Srober, W., *Currents Protocols in Immunology* 1993, Greene, Editions Wiley - Interscience).

### X - Conception de nouveaux vaccins à partir de la protéine p35.

Afin de déterminer si la protéine p35 ou une partie de celle-ci pouvait être utilisée comme vaccin pour protéger des souris de la souche BALB/c qui sont susceptibles à l'infection par le parasite *Leishmania major,* la protéine p24 a été produite en grande quantité (culture de 500 ml) à partir des bactéries du clone 23.12.10.33 et purifiée sur des colonnes d'affinité (résine Ni-NTA).

La protéine recombiante obtenue était pure à plus de 99% (analyse par électrophorèse à travers un gel de polyacrylamide (SDS-PAGE)).

En parallèle, une protéine recombinante contrôle (ovalbumine de poulet) a été produite et purifiée dans les mêmes conditions.

La protéine p24 ainsi que la protéine ovalbumine contrôle ont ensuite été administrées en parallèle à des souris de la souche BALB/c selon un protocole décrit par Afonso, L.C.C. et al. (Afonso, L.C.C., Scharton, T.M., Vieira, L.Q., Wysocka, M., Trinchieri, G., Scott, P., 1993 *Science,* vol. 263, pages 235-237).

Des quantités variables de protéines recombinantes (entre 100 ng et 50 µg) ont été injectées dans les coussinets plantaires gauches de souris BALB/c agées de 6 à 8 semaines en présence (1 µg) ou en abscence d'interleukine-12. Dix jours plus tard, ces mêmes souris ont reçu une injection intradermique de la protéine p24 ou de la protéine contrôle avec ou sans interleukine-12. Deux semaines plus tard, les souris ont été infectées par injection intradermique de 10⁵ promastigotes métacycliques du parasite *Leishmania major.* Dix semaines après l'infection, la moyenne des lésions des souris immunisées avec la protéine p24 en présence d'interleukine-12 était de 0.2 mm alors que celles des souris immunisées avec la protéine recombinante contrôle en présence ou en abscence d'interleukine-12 était de 4 mm. De même, la moyenne de la lésion des souris n'ayant reçu qu'une injection d'interleukine-12 était de 5 mm.

La protéine p35 représente le premier antigène immunodominant du parasite *Leishmania major* mis en évidence à ce jour

Les travaux ayant donné lieu à la présente invention permettent de produire cet antigène sous la forme d'une protéine recombinante par exemple dans *Escherichia coli* ce qui élimine les risques de contamination par d'autres protéines parasitaires. En outre, ces travaux offrent la possibilité de cloner le gène correspondant à tout ou partie de la protéine p35 dans des vecteurs pouvant être utilisés pour la vaccination de populations humaines ou animales (BCG, vecteur vaccine) et plus particulièrement :
- les populations humaines à risque dans les pays où la leishmaniose existe à l'état endémique comme le Soudan en Afrique centrale ou le Brésil en Amérique du Sud;
- les individus à risque dans les régions du monde occidental où l'on observe une recrudescence des cas survenant chez l'adulte (Alpes Maritimes en France);
- les animaux comme les chiens qui constituent des réservoirs du parasite dans des pays développés, en particulier dans le sud de la France, dans la région Provence-Côte d'Azur.

### LISTE DE SEQUENCES

### INFORMATION POUR LA SEQ ID NO : 1 :

### CARACTERISTIQUES DE LA SEQUENCE

| | |
|---|---|
| A) LONGUEUR : | 1070 paires de bases et 312 acide aminés |
| B) TYPE : | acide nucléique et protéine correspondante |
| C) NOMBRE DE BRIN: | ADN double brin |
| D) CONFIGURATION : | linéaire |
| TYPE DR MOLECULE : | ADNc |
| HYPOTHETIOUE : | non |
| ANTI-SENS : | non |

| ORIGINE | |
|---|---|
| A) ORGANISME : | Leishmania major |
| B) SOUCHE : | Wordl Health Organisation strain WHOM/IR/-/1 |
| D) STADE DE DEVELOPPEMENT : | promastigote |
| CARACTERISTIOUE ADDITIONNELLE | |
| A) NOM/CLE : | protéine p35 |
| B) EMPLACEMENT : | - de l'acide aminé 1 |
| | - à l'acide aminé 312 |
| D) AUTRES INFORMATIONS IMPORTANTES: | séquence codante pour la protéine p35 |
| | - du nucléotide 72 |
| | - au nucléotide 1010 |

| CARACTERISTIOUE ADDITIONNELLE | |
|---|---|
| A) NOM/CLE : | protéine p24 |
| B) EMPLACEMENT : | - de l'acide aminé 142 |
| | - à l'acide aminé 312 |
| D) AUTRES INFORMATIONS IMPORTANTES : | séquence codante pour la protéine p24 |
| | - du nucléotide 495 |
| | - au nucléotide 1010 |

| CARACTERISTIOUE ADDITIONNELLE | |
|---|---|
| A) NOM/CLE : | peptide de la protéine p24 |
| B) EMPLACEMENT: | - de l'acide aminé 156 |
| | - à l'acide aminé 169 |

### DESCRIPTION DE SEQUENCE : SEQ ID NO : 1 :

AACTAACGCT ATATAAGTAT CAGTTTCTGT ACTTTATTGA TAAGTTTCTT CTCACTCCAC 60

TTCGTTTCAC C ATG AAC TAC GAG GGT CAC CTG AAG GGT CAC CGA GGA TGG 110
Met Asn Tyr Glu Gly His Leu Lys Gly His Arg Gly Trp
1               5                   10

GTC ACC TCC CTG GCC TGC CCG CAG CAG GCG GGG TCG TAC ATC AAG GTG 158
Val Thr Ser Leu Ala Cys Pro Gln Gln Ala Gly Ser Tyr Ile Lys Val
    15                  20                  25

GTG TCG ACG TCG CGC GAT GGT ACG GTC ATC TCG TGG AAG GCC AAC CCC 206
Val Ser Thr Ser Arg Asp Gly Thr Val Ile Ser Trp Lys Ala Asn Pro
30                  35                  40                  45

GAC CGC CAC AGC TGG ACA GCG ACT ACG GTC TGT CGA ACC ACC GCC TCG 254
Asp Arg His Ser Trp Thr Ala Thr Thr Val Cys Arg Thr Thr Ala Ser
                50                  55                  60
AGG GGC ACA CCG GCT TCG TGT CGT GCG TGT CGC TGG GCC ACG CCA CCG 302
Arg Gly Thr Pro Ala Ser Cys Arg Ala Cys Arg Trp Ala Thr Pro Pro
            65                  70                  75

TAC TAC GCG CTG ACC GTG TCC TGG GAC CGC TCC ATC CGT ATG TGG GAC 350
Tyr Tyr Ala Leu Thr Val Ser Trp Asp Arg Ser Ile Arg Met Trp Asp
        80                  85                  90

CTG CGC ATT GGA CAG TGC CAG CGC AAG TTC CTG AAG CAC ACC AAG GAC 398
Leu Arg Ile Gly Gln Cys Gln Arg Lys Phe Leu Lys His Thr Lys Asp
    95                  100                 105

GTG CTC ACC GTC GCC TTC TCA CCG GAC GAC CGC CTG ATC GTG TCC GCG 446
Val Leu Thr Val Ala Phe Ser Pro Asp Asp Arg Leu Ile Val Ser Ala
110                 115                 120                 125

GGC CGC GAC AAC GTG ATC CGC GTG TGG AAC GTG GCG GGT GAG TGC ATG 494
Gly Arg Asp Asn Val Ile Arg Val Trp Asn Val Ala Gly Glu Cys Met
                130                 135                 140

CAC GAG TTC CTG CGC GAC GGT CAC GAG GAC TGG GTG AGC AGC ATC TGC 542
His Glu Phe Leu Arg Asp Gly His Glu Asp Trp Val Ser Ser Ile Cys
            145                 150                 155

TTC TCG CCG TCG CTG GAG CAC CCG ATC GTG GTG TCC GGC AGC TGG GAC 590
Phe Ser Pro Ser Leu Glu His Pro Ile Val Val Ser Gly Ser Trp Asp
        160                 165                 170

AAC ACC ATC AAA GTA TGG AAC GTG AAC GGG GGC AAG TGT GAG CGC ACG 638
Asn Thr Ile Lys Val Trp Asn Val Asn Gly Gly Lys Cys Glu Arg Thr
    175                 180                 185

CTC AAG GGC CAC AGT AAC TAC GTG TCC ACG GTG ACG GTG TCG CCA GAC 686
Leu Lys Gly His Ser Asn Tyr Val Ser Thr Val Thr Val Ser Pro Asp
190                 195                 200                 205

GGG TCT CTG TGC GCA TCT TGC GGC AAG GAC GGC GCG GTG CTG TTG TGG 734
Gly Ser Leu Cys Ala Ser Cys Gly Lys Asp Gly Ala Val Leu Leu Trp
                210                 215                 220

GAC CTG AGC ACC GGT GAG CAG CTG TTC AAG ATC AAC GTG GAG TCG GCC 782
Asp Leu Ser Thr Gly Glu Gln Leu Phe Lys Ile Asn Val Glu Ser Ala
            225                 230                 235

ATC AAC CAG ATC GGC TTC TCG CCC AAC CGC TTC TGG ATG TGC GTC GCG 830
Ile Asn Gln Ile Gly Phe Ser Pro Asn Arg Phe Trp Met Cys Val Ala
        240                 245                 250

ACG GAG AGG TCT CTG TCC GTG TAC GAC CTG GAG AGC AAG GCC GTG ATT 878
Thr Glu Arg Ser Leu Ser Val Tyr Asp Leu Glu Ser Lys Ala Val Ile
    255                 260                 265

GCG GAG CTG ACG CCG GAC GGC GCG AAG CCG TCG GAG TGC ATC TCC ATT 926
Ala Glu Leu Thr Pro Asp Gly Ala Lys Pro Ser Glu Cys Ile Ser Ile
270                 275                 280                 285

GCC TGG TCC GCC GAC GGC AAC ACT CTG TAC TCC GGC CAC AAG GAC AAC 974
Ala Trp Ser Ala Asp Gly Asn Thr Leu Tyr Ser Gly His Lys Asp Asn
                290                 295                 300

CTG ATC CGC GTG TGG TCC ATC TCC GAC GCC GAG TAA TGGCCGCGCG 1020
Leu Ile Arg Val Trp Ser Ile Ser Asp Ala Glu *
            305                 310

CTGCTCGTGC CGGACGGCGT GGTAGGCGAG GGAGAACGAG CGAGAAGTGA 1070

## Revendications

1. Protéine **caractérisée en ce qu'**elle comprend la séquence de 312 acides aminés représentée dans la SEQ ID NO:1 en annexe, ou une forme modifiée possédant au moins 40 % d'identité avec ladite séquence.

2. Protéine selon la revendication 1, **caractérisée en ce qu'**elle est capable d'induire une réponse immune dirigée contre le parasite Leishmanie chez un hôte animal lorsqu'elle est introduite chez ledit hôte seule ou conjuguée à une molécule porteuse.

3. Protéine selon l'une quelconque des revendications 1 ou 2, **caractérisée en ce qu'**elle comprend une séquence d'au moins 10 acides aminés consécutifs dans la SEQ ID NO:1 en annexe, ou une forme modifiée de celle-ci possédant au moins 40 % d'identité avec ladite séquence.

4. Protéine selon l'une quelconque des revendications 1 à 3, **caractérisée en ce qu'**elle comprend la séquence délimitée par les acides aminés situés aux positions 143 et 312 dans la SEQ ID NO:1 en annexe, ou une forme modifiée de celle-ci possédant au moins 40 % d'identité avec ladite séquence.

5. Protéine selon l'une quelconque des revendications 1 à 3, **caractérisée en ce qu'**elle comprend ou est constituée par la séquence peptidique de 18 acides aminés délimitée par les acides aminés situés aux positions 156 et 173 dans la SEQ ID NO:1 en annexe, ou une forme modifiée de celle-ci possédant au moins 40 % d'homologie avec ladite séquence.

6. Conjugué présentant des propriétés antigéniques, **caractérisé en ce qu'**il est constitué par une protéine selon l'une des revendication 1 à 5 couplée à une molécule porteuse.

7. Anticorps notamment monoclonal dirigé contre au moins une protéine selon l'une quelconque des revendications 1 à 5 ou un conjugué selon la revendication 6.

8. Acide nucléique codant pour une protéine selon l'une quelconque des revendications 1 à 5.

9. Acide nucléique selon la revendication 8 **caractérisé en ce qu'**il comprend la séquence nucléotidique représentée dans la SEQ ID NO:1 en annexe, ou une forme modifiée ou une partie de celle-ci dès lors qu'elle est capable de s'hybrider avec ladite séquence ou avec son complémentaire.

10. Acide nucléique selon l'une quelconque des revendications 8 à 9, **caractérisé en ce qu'**il comprend la séquence délimitée par les nucléotides situés aux positions 72 et 1010 dans la SEQ ID NO:1 en annexe, ou une forme modifiée ou une partie de celle-ci dès lors qu'elle est capable de s'hybrider avec ladite séquence ou avec son complémentaire.

11. Acide nucléique selon l'une quelconque des revendications 8 à 10, **caractérisé en ce qu'**il comprend la séquence délimitée par les nucléotides situés aux positions 498 et 1010 dans la SEQ ID NO:1 en annexe, ou une forme modifiée ou une partie de celle-ci dès lors qu'elle est capable de s'hybrider avec ladite séquence ou avec son complémentaire.

12. Sonde nucléotidique constituée d'un acide nucléique selon l'une quelconque des revendications 8 à 11 ou de son complémentaire, éventuellement marqué.

13. Acide nucléique recombinant, **caractérisé en ce qu'**il comprend un acide nucléique selon l'une quelconque des revendications 8 à 11 associé avec un promoteur et/ou un terminateur de transcription reconnu par les enzymes de la cellule hôte dans laquelle ledit acide nucléique recombinant est introduit.

14. Vecteur recombinant, **caractérisé en ce qu'**il comporte un insert d'acide nucléique selon l'une quelconque des revendications 8 à 11.

15. Cellule eucaryote ou procaryote isolée, **caractérisée en ce qu'**elle incorpore un acide nucléique recombinant selon la revendication 13 ou un vecteur recombinant selon la revendication 14.

16. Virus recombinant, **caractérisé en ce qu'**il incorpore un acide nucléique recombinant selon la revendication 13.

17. Composition antigénique comprenant à titre d'agent actif au moins une protéine selon l'une quelconque des revendications 1 à 5 et/ou un conjugué selon la revendication 6.

18. Méthode de dépistage *in vitro* de la leishmaniose **caractérisée en ce qu'**elle comprend :
- la mise en contact d'au moins un anticorps selon la revendication 7 avec un prélèvement biologique d'un sujet susceptible d'être infecté par un parasite responsable de leishmaniose, dans des conditions permettant la formation d'un complexe immunologique entre ledit anticorps et les protéines antigéniques contenues dans le prélèvement;
- la détection dudit complexe par tout moyen approprié.

19. Trousse de diagnostic pour la mise en oeuvre de la méthode selon la revendication 18, **caractérisé en ce qu'**elle comprend :
- une quantité déterminée d'au moins un anticorps selon la revendication 7,
- un milieu approprié à la formation de complexes immunologiques avec ledit anticorps,
- des réactifs permettant la détection des complexes éventuellement formés,
- éventuellement des échantillons témoins.

20. Méthode de dépistage *in vitro* de la leishmaniose **caractérisée en ce qu'**elle comprend :
- la mise en contact d'au moins une protéine selon l'une quelconque des revendications 1 à 5 ou d'un conjugué selon la revendication 6 ou d'une composition antigénique selon la revendication 17, avec un prélèvement biologique d'un sujet susceptible d'être infecté par un parasite responsable de leishmaniose, dans des conditions permettant la formation d'un complexe immunologique entre ladite protéine ou composition antigénique et les anticorps anti-p35 éventuellement présents dans le prélèvement analysé,
- la détection dudit complexe par tout moyen approprié.

21. Trousse de diagnostic pour la mise en oeuvre de la méthode selon la revendication 20, **caractérisée en ce qu'**elle comprend :
- une quantité déterminée d'au moins une protéine selon l'une quelconque des revendications 1 à 5
ou d'un conjugué selon la revendication 6 ou d'une composition antigénique selon la revendication 17,
- un milieu approprié à la formation de complexes immunologiques avec ladite protéine ou ladite composition antigénique,
- des réactifs permettant la détection des complexes éventuellement formés,
- éventuellement des échantillons témoins.

22. Composition pharmaceutique utile comme vaccin chez l'homme ou l'animal contre la leishmaniose, **caractérisée en ce qu'**elle comporte à titre d'agent actif au moins une protéine selon l'une des revendications 1 à 5 ou un virus recombinant vivant selon la revendication 16 éventuellement dans un véhicule pharmaceutiquement acceptable.

## Patentansprüche

1. Protein, **dadurch gekennzeichnet, dass** es die in der anliegenden SEQ ID NO:1 dargestellte Sequenz von 312 Aminosäuren aufweist oder eine modifizierte Form, die mit der besagten Sequenz zu mindestens 40 Prozent identisch ist.

2. Protein nach Anspruch 1, **dadurch gekennzeichnet, dass** es in der Lage ist, bei einem tierischen Wirt eine gegen den Parasiten Leishmania gerichtete Immunantwort zu bewirken, wenn es in den besagten Wirt allein oder in Verbindung mit einem Trägermolekül eingeführt wird.

3. Protein nach einem der Ansprüche 1 oder 2, **dadurch gekennzeichnet, dass** es eine Sequenz von mindestens 10 in der anliegenden SEQ ID NO:1 aufeinanderfolgenden Aminosäuren aufweist oder eine modifizierte Form dieser, die mit der besagten Sequenz zu mindestens 40 Prozent identisch ist.

4. Protein nach einem der Ansprüche 1 bis 3, **dadurch gekennzeichnet, dass** es die Sequenz aufweist, die von den Aminosäuren begrenzt ist, die sich an den Positionen 143 und 312 in der anliegenden SEQ ID NO:1 befinden, oder eine modifizierte Form dieser, die mit der besagten Sequenz zu mindestens 40 Prozent identisch ist.

5. Protein nach einem der Ansprüche 1 bis 3, **dadurch gekennzeichnet, dass** es die Peptidsequenz von 18 Aminosäuren aufweist oder aus dieser besteht, die von den Aminosäuren begrenzt ist, die sich an den Positionen 156 und 173 in der anliegenden SEQ ID NO:1 befinden, oder eine modifizierte Form dieser, die mit der besagten Sequenz zu mindestens 40 Prozent identisch ist.

6. Konjugat mit antigenen Eigenschaften, **dadurch gekennzeichnet, dass** es von einem Protein nach einem der Ansprüche 1 bis 5 gebildet wird, das an ein Trägermolekül gekoppelt ist.

7. Insbesondere monoklonaler Antikörper, der mindestens gegen ein Protein nach einem der Ansprüche 1 bis 5 oder ein Konjugat nach Anspruch 6 gerichtet ist.

8. Ein Protein kodierende Nukleinsäure nach einem der Ansprüche 1 bis 5.

9. Nukleinsäure nach Anspruch 8, **dadurch gekennzeichnet, dass** sie die in der anliegenden SEQ ID NO:1 dargestellte Nukleotidsequenz aufweist oder eine modifizierte Form oder einen Teil dieser, infolgedessen sie in der Lage ist, mit der besagten Sequenz oder mit ihrem Komplement Hybride zu bilden.

10. Nukleinsäure nach einem der Ansprüche 8 bis 9, **dadurch gekennzeichnet, dass** sie die Sequenz aufweist, die von den Nukleotiden begrenzt wird, die sich an den Positionen 72 und 1010 in der anliegenden SEQ ID NO:1 befinden, oder eine modifizierte Form oder einen Teil dieser, infolgedessen sie in der Lage ist, mit der besagten Sequenz oder ihrem Komplement Hybride zu bilden.

11. Nukleinsäure nach einem der Ansprüche 8 bis 10, **dadurch gekennzeichnet, dass** sie die Sequenz aufweist, die von den Nukleotiden begrenzt wird, die sich an den Positionen 498 und 1010 in der anliegenden SEQ ID NO:1 befinden, oder eine modifizierte Form oder einen Teil dieser, infolgedessen sie in der Lage ist, mit der besagten Sequenz oder ihrem Komplement Hybride zu bilden.

12. Nukleotidsonde, bestehend aus einer Nukleinsäure nach einem der Ansprüche 8 bis 11 oder ihrem eventuell markierten Komplement.

13. Rekombinante Nukleinsäure, **dadurch gekennzeichnet, dass** sie eine Nukleinsäure nach einem der Ansprüche 8 bis 11 aufweist, die mit einem Transkriptionspromotor und/oder einem -terminator verbunden ist, der von den Enzymen der Wirtszelle erkannt wird, in welche die besagte neukombinierte Nukleinsäure eingeschleust wurde.

14. Rekombinanter Vektor, **dadurch gekennzeichnet, dass** er ein Nukleinsäureinsert nach einem der Ansprüche 8 bis 11 aufweist.

15. Isolierte eukaryontische oder prokaryontische Zelle, **dadurch gekennzeichnet, dass** sie eine rekombinante Nukleinsäure nach Anspruch 13 oder einen rekombinanten Vektor nach Anspruch 14 inkorporiert.

16. Rekombinanter Virus, **dadurch gekennzeichnet, dass** er eine rekombinante Nukleinsäure nach Anspruch 13 inkorporiert.

17. Antigene Zusammensetzung, die als aktiven Wirkstoff mindestens ein Protein nach einem der Ansprüche 1 bis 5 und/oder ein Konjugat nach Anspruch 6 aufweist.

18. Methode zur *In-vitro*-Erkennung der Leishmaniose, **dadurch gekennzeichnet, dass** diese aufweist:
- die Zusammenführung von mindestens einem Antikörper nach Anspruch 7 mit einer biologischen Probe einer Versuchsperson, die von einem Leishmaniose auslösenden Parasiten infiziert sein könnte, unter Bedingungen, unter denen sich ein immunologischer Komplex aus dem besagten Antikörper und den antigenen Proteinen, die in der Probe enthalten sind, bilden kann;
- der Nachweis des besagten Komplexes durch jedes geeignete Mittel.

19. Diagnoseset zur Durchführung der Methode nach Anspruch 18, **dadurch gekennzeichnet, dass** dieses aufweist:
- eine bestimmte Menge mindestens eines Antikörpers nach Anspruch 7,
- ein Milieu, das für die Bildung von immunologischen Komplexen mit dem besagten Antikörper geeignet ist,
- Reagenzien, mit denen eventuell gebildete Komplexe nachgewiesen werden können,
- eventuell Vergleichsmuster.

20. Methode zur *In-vitro*-Erkennung der Leishmaniose, **dadurch gekennzeichnet, dass** diese aufweist:
- die Zusammenführung von mindestens einem Protein nach einem der Ansprüche 1 bis 5 oder eines Konjugats nach Anspruch 6 oder einer antigenen Zusammensetzung nach Anspruch 17 mit einer biologischen Probe einer Versuchsperson, die von einem Leishmaniose auslösenden Parasiten infiziert sein könnte, unter Bedingungen, unter denen sich ein immunologischer Komplex aus dem besagten Protein oder der antigenen Zusammensetzung und den Antikörpern anti-p35, die eventuell in der analysierte Probe enthalten sind, bilden kann,
- der Nachweis des besagten Komplexes durch jedes geeignete Mittel.

21. Diagnoseset zur Durchführung der Methode nach Anspruch 20, **dadurch gekennzeichnet, dass** dieses aufweist:
- eine bestimmte Menge mindestens eines Proteins nach einem der Ansprüche 1 bis 5 oder eines Konjugats nach Anspruch 6 oder einer antigenen Zusammensetzung nach Anspruch 17,
- ein Milieu, das für die Bildung von immunologischen Komplexen mit dem besagten Protein oder der besagten antigenen Zusammensetzung geeignet ist,
- Reagenzien, mit denen eventuell gebildete Komplexe nachgewiesen werden können,
- eventuell Vergleichsmuster.

22. Pharmazeutische Zusammensetzung, die als Leishmaniose-Impfstoff bei Mensch oder Tier dient, **dadurch gekennzeichnet, dass** sie als aktiven Wirkstoff mindestens ein Protein nach einem der Ansprüche 1 bis 5 oder einen rekombinanten lebenden Virus nach Anspruch 16 aufweist, eventuell in einem pharmazeutisch akzeptablen Arzneistoffträger.

## Claims

1. Protein **characterised in that** it comprises the sequence of 312 amino acids represented in SEQ ID No. 1 in the appendix or a modified form with at least 40% identical with the said sequence.

2. Protein according to claim 1, **characterised in that** it is capable of inducing an immune response directed against the Leishmania parasite in the host animal when it is introduced in the said host alone or combined with a carrier molecule.

3. Protein according to either of claims 1 and 2, **characterised in that** it comprises a sequence of at least 10 consecutive amino acids in SEQ ID No. 1 in the appendix or a modified form of this sequence with at least 40% identical with the said sequence.

4. Protein according to any one of claims 1 to 3, **characterised in that** it comprises the sequence delimited by the amino acids located at positions 143 and 312 in SEQ ID No. 1 in the appendix, or a modified form of this sequence with at least 40% identical with the said sequence.

5. Protein according to any one of claims 1 to 3, **characterised in that** it comprises or is composed of the peptidic sequence of 18 amino acids delimited by the amino acids located at positions 156 and 173 in SEQ ID No. 1 in the appendix, or a modified form of this sequence with at least 40% corresponding to the said sequence.

6. Conjugate with antigenic properties, **characterised in that** it is composed of a protein according to one of claims 1 to 5 coupled with a carrier molecule.

7. An antibody, and particularly a monoclonal antibody directed against at least one protein according to any one of claims 1 to 5 or a conjugate according to claim 6.

8. Coding nucleic acid for a protein according to any one of claims 1 to 5.

9. Nucleic acid according to claim 8, **characterised in that** it comprises the nucleotidic sequence represented in SEQ ID No. 1 in the appendix, or a modified form or part of this sequence provided that it is capable of hybridising with the said sequence or with its complement.

10. Nucleic acid according to either of claims 8 and 9, **characterised in that** it comprises the sequence delimited by the nucleotides located at positions 72 and 1010 in SEQ ID No. 1 in the appendix, or a modified form or part of this sequence provided that it is capable of hybridising with the said sequence or with its complement.

11. Nucleic acid according to any one of claims 8 to 10, **characterised in that** it comprises the sequence delimited by nucleotides located at positions 498 and 1010 in SEQ ID No. 1 in the appendix, or a modified form or part of this sequence provided that it is capable of hybridising with the said sequence or with its complement.

12. Nucleotidic probe composed of a nucleic acid according to any one of claims 8 to 11 or its complement, possibly marked.

13. Recombining nucleic acid, **characterised in that** it comprises a nucleic acid according to any one of claims 8 to 11 associated with a promoter and / or a transcription terminator recognised by the enzymes of the host cell in which the said recombining nucleic acid is introduced.

14. Recombining vector, **characterised in that** it comprises a nucleic acid insert according to any one of claims 8 to 11.

15. Isolated eukaryote or prokaryote cell, **characterised in that** it incorporates a recombining nucleic acid according to claim 13 or a recombining vector according to claim 14.

16. Recombining virus, **characterised in that** it incorporates a recombining nucleic acid according to claim 13.

17. Antigenic composition comprising at least one protein according to any one of claims 1 to 5 and / or a conjugate according to claim 6 as active agent.

18. *In vitro* tracking method for leishmaniasis **characterised in that** it comprises:
- bringing at least one antibody according to claim 7 into contact with a biological sample taken from a subject that might by infected by a parasite responsible for leishmaniasis, under conditions enabling the formation of an immunological complex between the said antibody and the antigenic proteins contained in the sample;
- detection of the said complex by any appropriate means.

19. Diagnostic kit for use of the method according to claim 18, **characterised in that** it comprises:
- a determined quantity of at least one antibody according to claim 7,
- a medium appropriate to the formation of immunological complexes with the said antibody,
- reagents for the detection of any complexes that are formed,
- possibly control samples.

20. *In vitro* tracking method for leishmaniasis, **characterised in that** it comprises:
- bringing at least one protein according to any one of claims 1 to 5 or a conjugate according to claim 6 or an antigenic composition according to claim 17, into contact with a biological sample taken from a subject that might by infected by a parasite responsible for leishmaniasis, under conditions enabling the formation of an immunological complex between the said antibody and the antigenic proteins contained in the sample;
- detection of the said complex by any appropriate means.

21. Diagnostic kit for use of the method according to claim 20, **characterised in that** it comprises:
- a determined quantity of at least one protein according to any one of claims 1 to 5 or a conjugate according to claim 6 or an antigenic composition according to claim 17,
- a medium conducive to the formation of immunological complexes with the said protein or the said antigenic composition,
- reagents for the detection of any complexes that are formed,
- possibly control samples.

22. Pharmaceutical composition useful as a vaccine in man or animal against leishmaniasis, **characterised in that** it comprises at least one protein according to one of claims 1 to 5 or a living recombining virus according to claim 16, possibly in a pharmaceutically acceptable vehicle, as its active agent.
